(19) Europäisches Patentamt
European Patent Office
Office européen des brevets

(11) EP 0 706 320 B1

(12) EUROPEAN PATENT SPECIFICATION

(45) Date of publication and mention of the grant of the patent:
**08.01.1997 Bulletin 1997/02**

(21) Application number: **94921398.7**

(22) Date of filing: **24.06.1994**

(51) Int. Cl.$^6$: **A01N 63/00**

(86) International application number:
**PCT/US94/07252**

(87) International publication number:
**WO 95/01098 (12.01.1995 Gazette 1995/03)**

# (54) METHOD OF CONTROLLING INSECTS

VERFAHREN ZUM BEKÄMPFEN VON INSEKTEN

METHODE DE CONTROLE DE LA PROLIFERATION DES INSECTES

(84) Designated Contracting States:
**AT BE CH DE DK ES FR GB GR IE IT LI LU MC NL PT SE**

(30) Priority: **28.06.1993 US 83948**

(43) Date of publication of application:
**17.04.1996 Bulletin 1996/16**

(73) Proprietor: **MONSANTO COMPANY**
**St. Louis Missouri 63167 (US)**

(72) Inventors:
- **CORBIN, David, Richard**
  **Chesterfield, MO 63017 (US)**
- **GREENPLATE, John, Thomas**
  **Manchester, MO 63021 (US)**
- **JENNINGS, Michael Girard**
  **Chesterfield, MO 63017 (US)**
- **PURCELL, John, Patrick**
  **Ballwin, MO 63011 (US)**
- **SAMMONS, Robert, Douglas**
  **New Melle, MO 63365 (US)**

(74) Representative: **Nash, Brian Walter et al**
**Monsanto Services International S.A.**
**Patent Department,**
**Avenue de Tervuren 270/272,**
**Letter Box No. 21**
**B-1150 Bruxelles (BE)**

(56) References cited:
- **BIOCHEMICAL AND BIOPHYSICAL RESEARCH COMMUNICATIONS, vol.196, no.3, 15 November 1993, NEW YORK pages 1406 - 1403 J.P. PURCELL ET AL. 'Cholesterol oxidase: a potent insecticidal protein active against boll weevil larvae.'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 706 320 B1

## Description

### FIELD OF THE INVENTION

This invention relates to a method of controlling lepidopteran insects by use of an enzyme which may be applied directly to the plant or produced thereon by microorganisms or by genetically modifying the plant to produce the enzyme, and to genes, microorganisms, and plants useful in that method.

### BACKGROUND OF THE INVENTION

The use of natural products, including proteins, is a well known method of controlling many insect pests. For example, endotoxins of *Bacillus thuringiensis (B.t.)* are used to control both lepidopteran and coleopteran insect pests. Genes producing these endotoxins have been introduced into and expressed by various plants, including cotton, tobacco, and tomato. There are, however, several economically important insect pests that are not susceptible to *B.t.* endotoxins. There is also a need for additional proteins which control insects for which *B.t.* provides control in order to manage any development of resistance in the population.

It is therefore an object of the present invention to provide proteins capable of controlling lepidopteran insects and genes useful in producing such proteins. It is a further object of the present invention to provide genetic constructs for and methods of inserting such genetic material into microorganisms and plant cells. It is another object of the present invention to provide transformed microorganisms and plants containing such genetic material.

### SUMMARY OF THE INVENTION

It has been discovered that proteins that catalyze the oxidation of 3-hydroxysteroids, for example, cholesterol, will control lepidopteran insects. They cause mortality and stunting of larvae of lepidopteran insects. The enzymes may be applied directly to plants or introduced in other ways such as through the application of plant-colonizing microorganisms or by the plants themselves, which have been transformed to produce the enzymes.

3-Hydroxysteroid oxidases (E.C.1.1.3.6) are naturally produced by microorganisms such as *Streptomyces* sp., *Pseudomonas* sp., *Mycobacterium* sp., *Schizophyllum commune*, *Nocardia* sp., and *Rhodococcus* sp. [Smith et al., 1976, and Long et al., 1990.]. Preparations of enzymes from several different sources are available from Sigma Chemical Company, St. Louis, Missouri.

New *Streptomyces* genes that control the expression of 3-hydroxysteroid oxidase have been isolated and sequenced. These new genes or genes from other known producers of 3-hydroxysteroid oxidase may be inserted into a transformation vector cassette which is used to transform plant-colonizing microorganisms which when applied to plants express the genes producing a 3-hydroxysteroid oxidase, thereby providing control of insects. Alternatively, genes which function in plants and encode the subject enzymes may be inserted into transformation vector cassettes which may be incorporated into the genome of the plant, which then protects itself from attack by expressing the gene and producing a 3-hydroxysteroid oxidase. Additionally, the plant may also be transformed to co-express *B.t.* genes which express proteins for the control of other insects. Examples of plants transformed to express *B.t.* genes are disclosed in European Patent Publication No. 0 385 962 (Fischhoff et al.).

In accomplishing the foregoing, there is provided, in accordance with one aspect of the present invention, a method of controlling insect infestation of plants comprising providing a 3-hydroxysteroid oxidase for ingestion by the insect.

In accordance with another aspect of the present invention, there is provided a method of producing genetically transformed plants which express an amount of a 3-hydroxysteroid oxidase effective to control lepidioteran insects, comprising the steps of:

a) inserting into the genome of a plant cell a recombinant, double-stranded DNA molecule comprising

(i) a promoter which functions in plant cells to cause the production of an RNA sequence;
(ii) a structural coding sequence that encodes for 3-hydroxysteroid oxidase; and
(iii) a 3' non-translated region which functions in said plant cells to cause the addition of polyadenylate nucleotides to the 3' end of the RNA sequence,

wherein said promoter is heterologous with respect to the structural coding sequence and wherein said promoter is operatively linked with said structural coding sequence, which is in turn operably linked with said non-translated region;
b) obtaining transformed plant cells; and
c) regenerating from the transformed plant cells genetically transformed plants which express an insecticidally effective amount of sterol oxidase.

There is also provided, in accordance with another aspect of the present invention, bacterial and transformed plant cells that contain DNA comprised of the above-mentioned elements (i), (ii), and (iii).

As used herein, the term "controlling insect infestation" means reducing the number of insects which cause reduced yield, either through mortality, retardation of larval development (stunting), or reduced reproductive efficiency.

As used herein, the term "structural coding sequence" means a DNA sequence which encodes for a polypeptide, which may be made by a cell following transcription of the DNA to mRNA, followed by translation to the desired polypeptide.

DETAILED DESCRIPTION OF THE INVENTION

3-Hydroxysteroid oxidases catalyze the oxidation of the 3-hydroxy group of 3-hydroxysteroids to produce ketosteroids and hydrogen peroxide. They are capable of catalyzing the oxidation of various 3-hydroxysteroids, such as, for example, cholesterol. Most of the previously known 3-hydroxysteroid oxidases are called "cholesterol oxidases" (enzymatically catalogued as E.C. #1.1.3.6) but cholesterol is only one of the 3-hydroxysteroid substrates. The use of all 3-hydroxysteroid oxidases and the genes encoding such proteins, for the purpose of controlling insects, are within the scope of the present invention.

3-Hydroxysteroid oxidases are commercially available for use as reagents for serum cholesterol assays. For example, Sigma Chemical Company, St. Louis, Missouri, offers three such 3-hydroxysteroid oxidases (denominated as cholesterol oxidases), one from a *Streptomyces* sp., one from a *Pseudomonas fluorescens*, and one from a *Brevibacterium*. Two other sources of 3-hydroxysteroid oxidase, two streptomycetes denominated A19241 and A19249, each of which produce a 3-hydroxysteroid oxidase, have been isolated. The organisms were collected in Madagascar. When these organisms were cultured according to usual methods the culture filtrates were found to affect insect larvae as described below.

A seed culture of A19249 was started in 55 mL sterile Tryptone-Yeast Extract broth, pH 6.8, in a 250 mL shake flask. The seed was agitated at 250 rpm on a rotary shaker for 3 days at 30 °C. A New Brunswick Bioflo II Bioreactor with a 2 L working volume was filled with "medium 202" ($MgSO_4 \cdot 7H_2O$ 2 g/L, $KH_2PO_4$ 0.5 g/L, NaCl 0.5 g/L, $CaCO_3$ 1 g/L, $ZnSO_4 \cdot H_2O$ (1 mg/mL stock) 5 mL/L, 100 mM FeEDTA 0.5 mL/L, Soluble Starch 5 g/L, Dextrose 2.5 g/L, Malt Extract 2.5 g/L, Soytone 5 g/L). The pH was adjusted to 6.5 with 2.5 N NaOH or 1 N HCl, and 1 mL/L of P2000, an antifoam agent was added. The bioreactor was sealed and autoclaved for 25 min at 250 °C. The seed, at 3 days growth, was used to inoculate the fermentor at 2% or 40 mL. The fermentation took place at 30 °C with an airflow of 1 L/min and agitation running at 500 rpm. The fermentation was harvested after 40 h.

Each of these enzymes has demonstrated control of insects as shown below. The *P. fluorescens* 3-hydroxysteroid oxidase is immunologically distinct from the *Streptomyces* enzymes, but it also controls insects.

Other organisms producing 3-hydroxysteroid oxidases of the present invention may be identified by assaying culture filtrates or individual proteins for 3-hydroxysteroid oxidase activity using a spectrophotometric assay, described below, which measures hydrogen peroxide production in the presence of a 3-hydroxysteroid, for example, cholesterol [Gallo, 1981].

**BIOEFFICACY ASSAYS**

Larval Bioassay

Lepidopteran larvae were tested on artificial agar-based diet (Marrone et al., 1985) treated with the indicated amount of the A19249 3-hydroxysteroid oxidase (cholesterol oxidase) for six days. The results are shown in Table 1. Percent stunting is defined as

$$\frac{\text{larval weights (Herpes control) - larval weights (cholesterol oxidase)}}{\text{larval weights (Herpes control)}} \text{x100\%}$$

An extended test was performed with tobacco budworm larvae to test the effect of the stunting noted in the six-day test. Tobacco budworm eggs were added to artificial diet (as described above) containing either buffer or 100 ppm A19249 3-hydroxysteroid oxidase (cholesterol oxidase). After seven days, some mortality as compared to the controls was noted. Surviving larvae were moved to fresh diet (control or treated, as appropriate) every seven days. Percent mortality (corrected for control mortality) is reported for the 7, 10, and 14 day periods in Table 2. The corrected number of larvae was 23. By the 27th day only two larvae were alive; both were very stunted and never pupated. 92% of the control larvae survived and all survivors pupated by day 27. This experiment demonstrates that 3-hydroxysteroid oxidase essentially arrests the development of tobacco budworm larvae.

Table 1

| Insect | Stage | Dose-µg/mL | Stunting |
|---|---|---|---|
| tobacco budworm | egg/lv | 30 | 0 |
| | lv | 100 | 86% |
| corn earworm | lv | 50 | 0 |
| | lv | 100 | 35% |
| fall army worm | lv | 30 | 0 |
| tobacco hornworm | lv | 30 | 0 |
| | lv | 100 | 30% |
| pink bollworm | lv | 50 | 0 |
| | lv | 100 | 30% |
| European cornborer | lv | 50 | 0 |
| | lv | 100 | 46% |
| beet armyworm | lv | 100 | 76% |
| black cutworm | lv | 100 | 68% |

Table 2

| Interval (days) | Percent Mortality |
|---|---|
| 7 | 20 |
| 10 | 61 |
| 14 | 80 |

**MODE OF ACTION STUDIES**

The following studies show that 3-hydroxysteroid oxidase has a direct effect on the insect itself and that the activity demonstrated in the experiments described above cannot be attributed to the enzymes effect on the insect's diet, for example by sterol depletion. Boll weevil and lepidopteran larvae are most susceptible to the enzyme. It is believed that this specificity is due to the effect of 3-hydroxysteroid oxidase on the midgut of the insect as explained in more detail below. Other insects with similar midgut physiologies may also be controlled by 3-hydroxysteroid oxidase. In addition, 3-hydroxysteroid oxidases other than those tested and reported herein may control a different spectrum of insects with different midgut physiologies.

Cotton Seed Diet Assay

Treatment diet was made by mixing 30 g of Pharmamedia™ (Traders Protein) cottonseed flour into 170 mL of a 1.6% agar solution at 50 °C, containing 0.13% propionic acid, 0.014% phosphoric acid, and 30 mg each of streptomycin sulfate and chlor-tetracycline. Before mixing, 10% KOH was used to adjust the pH to 6.2. Pharmamedia™ is a flour made up of cottonseed embryos. The diet was incubated in a water bath at 40 °C. Dilutions of the Sigma *Streptomyces* 3-hydroxysteroid oxidase (cholesterol oxidase) were incorporated into the diets. Tobacco budworm larvae are 68% stunted when exposed to 3-hydroxysteroid oxidase (100 ppm) in cottonseed diet.

Cotton Callus Diet Assay

Cotton callus assays were conducted using Coker 312 cotton callus and a 96 well insect diet tray. Each well contained 0.5 ml of gelled 2% agar (containing 0.13% propionic acid and 0.014% phosphoric acid) that was covered with a filter paper disc. A piece of callus (approximately 150 mg) was placed in each well. For each replicate, 25 µl of a stock solution of 3-hydroxysteroid oxidase (1.25 mg/ml stock) or 25 µl of 25 mM Hepes buffer (pH 7.5) was pipetted onto each piece of callus. Tobacco budworm eggs (4-8 eggs/well, 0-12 hr from hatching) in 0.15% agar were pipetted onto the filter disc next to the callus. Individual larvae were transferred to freshly treated callus samples every three days. Larvae were weighed after 10 days.

Stunting of larvae was observed in the treated wells, although reduced as compared to the artificial diet or the cottonseed diet. This reduced level of stunting with cotton callus is probably due to the pipetted enzyme solution not completely covering the callus tissue and the loss of some enzyme onto the filter paper.

Pre-ingestion Effect on Diet

There are no lethal or stunting effects from feeding larvae a diet sample that was incubated for one week with a 3-hydroxysteroid oxidase and then heated to 80 °C to denature the enzyme prior to using it in the above assay. This further demonstrates that the mode of action of 3-hydroxysteroid oxidase is not dependent on sterol depletion of the nutrient source but that the enzyme is directly active upon the insect.

Microscopy Studies

Tobacco budworm larvae were reared from eggs on artificial diet containing 100 µg/ml of 3-hydroxysteroid oxidase or Hepes buffer control. After ten days, midgut epithelia were dissected and processed for microscopy as described by Purcell et al., 1993. The midguts from treated larvae exhibited a variety of histological and ultrastructural alterations as compared with Hepes buffer-treated controls. 3-Hydroxysteroid oxidase induced a lateral constriction and apical-basal elongation of the cells in the midgut epithelium, resulting in a widening of the inter-cellular spaces and the formation of an irregular and convoluted lumenal surface. The cellular attenuation was also characterized by localized apical cytoplasmic blebbing and microvillar denudation. This denudation occasionally generated detached cytoplasmic fragments in the lumen. Localized cytolysis was observed, but the epithelium as a whole remained intact. These observations suggest that exposure to 3-hydroxysteroid oxidase compromises the integrity of the apical plasma membrane which results in a secondary generalized cellular hypersensitivity to osmotic swelling and lysis.

Cholesterol is required for the integrity and normal function of virtually all cellular membranes. A reduction in the availability of cholesterol for incorporation into and maintenance of cellular membranes due to metabolism by 3-hydroxysteroid oxidase could therefore be one possible mechanism for toxicity of this agent. However, the observations described above show the most dramatic structural alterations are localized to the tips of the midgut cells in contact with the 3-hydroxysteroid oxidase in the gut lumen. This suggests that 3-hydroxysteroid oxidase may act directly to alter cholesterol already incorporated into the membrane. In addition, diet treatment studies described above demonstrated that the diet was not altered prior to ingestion in a manner which compromises tobacco budworm growth, again suggesting a direct effect of the enzyme on the insect.

Mode of Action Theory

While not being bound by this theory, it is believed that the 3-hydroxysteroid oxidase enzyme stunts the growth of lepidopteran larvae by some action in the gut after ingestion. The bioassay and morphological data show that there was clearly a pathological condition resulting from ingestion of diet containing 3-hydroxysteroid oxidase.

**ENZYME IDENTIFICATION**

The active proteins from the Madagascar *Streptomyces* microorganisms were isolated, purified, partially sequenced, and identified as 3-hydroxysteroid oxidases.

Protein Isolation

Each culture filtrate was purified by first sizing on YM10 membranes (Amicon) to a [>10 kDa] fraction, followed by multiple chromatography runs on an FPLC Mono Q HR10/10 (Pharmacia LKB, Piscataway, NJ) column. For chromatography on the Mono Q column, the samples were loaded on the column in 25 mM Hepes pH 7.5 and eluted with a gradient to 1.0 M KCl in 25 mM Hepes pH 7.5. Fractions were collected and aliquots of each were filtered through 0.2 µ Acrodisc syringe tip filters. Each was tested in an insect assay. Aliquots of insecticidally active fractions were electrophoresed on SDS-PAGE [Laemmli, 1970] using a Daiichi Double Gel Device and 10-20% mini-gel. Proteins were visualized by silver staining using Daiichi silver stain reagent kit. The active enzymes of the present invention, isolated from the novel microorganisms, were found to be a 52.5 kDa protein.

Amino Acid Sequences

An SDS-PAGE gel of the protein produced by *Streptomyces* A19241, isolated as above, was blotted onto PVDF paper (Immobilon, Millipore) using the protocol of Matsudaira [Matsudaira, 1987]. The N-terminus was sequenced using automated Edman degradation chemistry. A gas phase sequencer (Applied Biosystems, Inc.) was used for the degradation using the standard sequencer cycle. The respective PTH-aa derivatives were identified by reverse phase HPLC analysis in an on-line fashion employing a PTH analyzer (Applied Biosystems, Inc.) fitted with a Brownlee 2.1 mm i.d. PTH-C18 column. For internal sequences, digestions were carried out on purified 3-hydroxysteroid oxidase from A19249 using trypsin (TPCK-treated, from Worthington Biochemicals Corp., Freehold, NJ). Fragments were then purified by reverse phase HPLC and sequenced in an N-terminal fashion.

The resulting partial sequences were compared to known proteins and a strong (71%) homology was found with the reported fourteen amino acid sequence at the N-terminus of a 3-hydroxysteroid oxidase isolated from a *Streptomyces* species [Ishizaki et al., 1989]. The reported enzyme has an $M_r$ of 54.9 kDa which agrees well with the $M_r$ of 52.5 kDa of the isolated enzyme.

Six internal fragments of the purified enzyme from A19249, also having homology to six regions of the reported enzyme, were sequenced. The fragments had 95, 76, 64, 58, 89, and 100 percent sequence identities.

Amino Acid Composition Determination and Comparison

The amino acid composition of the 3-hydroxysteroid oxidase produced by A19249 was determined and compared with the composition of the reported *Streptomyces* enzyme. The samples were subjected to acid hydrolysis (6N HCl, vapor phase hydrolysis using a Water's Picotag workstation, 24 hr, 110 °C.). All analyses were performed after post-column derivation of the hydrolysates using ninhydrin [Moore et al., 1963]. A Beckman Model 6300 Auto analyzer was employed for the actual determinations. The S delta n/N statistic is used to compare two compositions in order to make a prediction about their relatedness. The formula for the statistic is:

$$1/2\ \Sigma\ (n_{Ai}-n_{Bi})^2/N$$

where A is one composition, B is the other composition, i is each amino acid, n is the number of each amino acid, N is the total number of amino acids in the protein. If S delta n/N is <0.42, then there is a greater than 95% chance that the proteins are related. The smaller the value, the more closely the determined compositions match.

The S delta n/N statistic for the A19249 protein compared to the reported enzyme is 0.36, indicating that the two are highly related.

3-Hydroxysteroid Oxidase Assay

The identity of the enzyme was confirmed by testing its ability to oxidize a 3-hydroxysteroid, specifically cholesterol. The enzyme is added to a reagent mixture comprising horseradish peroxidase (20 U/mL), phenol (14 mM), 4-amino antipyrine (0.82 mM), Triton® X-100 (0.05%) and phosphate buffer (100 mM, pH 7). The sterol in isopropanol is then added and the absorbance at 500 nm monitored. One unit of activity is defined as the amount of enzyme required to oxidize 1 µmole of sterol per minute at 20 °C.

The activity levels of the enzymes are reported in Table 12 for 3-hydroxysteroids representative of various classes of 3-hydroxysteroids. The enzyme sources are as follows:

1 = A19249
2 = A19241
3 = Sigma *Streptomyces*
4 = Sigma *Pseudomonas*

Table 3

| Sterol | Relative Rate for Enzymes | | | |
|---|---|---|---|---|
| | 1 | 2 | 3 | 4 |
| cholesterol | 100 | 100 | 100 | 100 |
| dihydrocholesterol | 56 | 56 | 59 | 69 |
| dehydrocholesterol | 13 | 12 | 7 | 47 |
| lathosterol | 28 | 34 | 27 | 71 |
| stigmasterol | 22 | 28 | 11 | 21 |
| sitosterol | 88 | 65 | 49 | 50 |
| campesterol* | 65 | 64 | 45 | 49 |
| fucosterol | 22 | 20 | 12 | 68 |
| lanosterol | <1 | <1 | <1 | 1 |
| ecdysone | <1 | <1 | <1 | <1 |
| 20-OH ecdysone | <1 | <1 | <1 | <1 |

*65/35 mixture of campesterol and dihydrobrassicasterol

<u>Immunological Comparison of Enzymes</u>

The Sigma *Streptomyces* enzyme is immunologically related to the 3-hydroxysteroid oxidases produced by the isolates of the present invention, numbers A19241 and 19249, as demonstrated by Western blotting [Burnette et al., 1981] using polyclonal antisera generated against the Sigma *Streptomyces* enzyme. The antisera recognized both enzymes produced by the isolates. The 3-hydroxysteroid oxidase from *P. fluorescens* was not recognized by the antisera. This demonstrates that immunologically distinct 3-hydroxysteroid oxidases are toxic to insects.

**GENETIC IDENTIFICATION**

The 3-hydroxysteroid oxidase gene was isolated from one of the *Streptomyces* microorganisms isolated in Madagascar and its sequence determined.

<u>Cloning of the 3-Hydroxysteroid Oxidase Gene from A19249</u>

As discussed above, peptide sequences of purified 3-hydroxysteroid oxidase from A19249 were obtained for four regions of the protein. These peptide sequences were compared to a database of known protein sequences, and this comparison revealed that the A19249 protein showed a high degree of homology to a known 3-hydroxysteroid oxidase from *Streptomyces* [Ishizaki]. Comparing the A19249 peptide sequences to this known protein sequence, these peptides were assigned to their likely positions in the A19249 protein sequence. The sequence derived from the intact 3-hydroxysteroid oxidase from A19249 corresponded to a region near the N-terminus of the secreted form of the enzyme from the published sequence. From this it was concluded that the A19249 N-terminal peptide sequence was also likely to correspond to a "mature" secreted form of the protein lacking its putative secretory signal sequence. This was later confirmed by the DNA sequence analysis of the A19249 gene (see below). Three peptides were used to construct hybridization probes for isolation of the A19249 3-hydroxysteroid oxidase gene. Peptide N2 (SEQ ID NO:1) corresponded to N-terminal amino acids 29 - 43 of the known mature protein sequence (sequence without the putative signal peptide); peptide C1 (SEQ ID NO:2) to amino acids 434 - 449 of the mature protein sequence; and peptide C2 (SEQ ID NO:3) to amino acids 464 - 475 of the mature protein sequence.

N2 (SEQ ID NO:1): ValSerThrLeuMetLeuGluMetGlyGlnLeuTrpAsnGlnPro
C1 (SEQ ID NO:2): AlaPheAlaAspAspPheCysTyrHisProLeuGlyGlyCysValLeu
C2 (SEQ ID NO:3): AsnLeuTyrValThrAspGlySerLeuIleProGly

Based on these peptide sequences, three long nondegenerate oligonucleotides, corresponding to 3-hydroxysteroid oxidase peptide sequences from A19249, were designed using *Streptomyces* preferred codons. The oligonucleotides N2 (SEQ ID NO:4), C1 (SEQ ID NO:5), and C2 (SEQ ID NO:6) correspond to the peptides N2, C1, and C2 described above.

N2 Probe (SEQ ID NO:4): gtgtccaccctgatgctggagatgggccagctgtggaaccagccc
C1 Probe (SEQ ID NO:5): gccttcgccgacgacttctgctaccacccgctcggcggctgcgtcctg
C2 Probe (SEQ ID NO:6): aacctctacgtgaccgacggttcgctgatcccgggt

Probes N2 (SEQ ID NO:4), C1 (SEQ ID NO:5), and C2 (SEQ ID NO:6) were all used as hybridization probes on Southern blots of A19249 genomic DNA. All three probes hybridized to the same 2.2 kb band in BamHI digested DNA, but N2 (SEQ ID NO:4) hybridized to a different fragment than C1 (SEQ ID NO:5) and C2 (SEQ ID NO:6) did in SalI and BglII digests. This indicated that SalI and BglII cut within the coding sequence of the 3-hydroxysteroid oxidase gene from A19249, which was confirmed by DNA sequence analysis.

The 3-hydroxysteroid oxidase gene from A19249 was isolated using the three synthetic oligonucleotides as hybridization probes on a library of DNA fragments of A19249 DNA in a lambda phage vector. A library was made in lambda EMBL3 using partial-digest Mbo1 DNA fragments of A19249. These probes were used to screen approximately 72,000 lamoda phage plaques from the primary library. Primary plaque screening was performed using N2 (SEQ ID NO:4) plus C2 (SEQ ID NO:6). A total of 12 recombinant plaques that hybridized to the N and C-terminal probes were picked and purified by a second round of hybridization screening with probes N2 (SEQ ID NO:4) and C2 (SEQ ID NO:6). Southern blot analysis revealed that, in five of six lambda clones analyzed, a 2.2 kb BamHI fragment hybridized to both the N and C-terminal probes. This result confirmed the earlier Southern hybridization analysis that indicated a 2.2 kb BamHI fragment contained the 3-hydroxysteroid oxidase gene. This 2.2 kb DNA fragment was cloned into plasmid vector pUC18 [Yanisch-Perron et al., 1985] in both orientations for further analysis. Restriction mapping showed that there were internal SalI and BglII sites as predicted by the Southern hybridization analysis. These sites are also conserved compared to the published 3-hydroxysteroid oxidase gene sequence. The BamHI fragment was further subcloned into four fragments for direct DNA sequencing.

Sequence Analysis of the 3-Hydroxysteroid Oxidase Gene

A total of 1865 nucleotides of DNA sequence from the 2.2 kb BamHI fragment were determined by direct DNA sequence analysis of subclones of this fragment using the dideoxy chain termination method. This sequence is identified as SEQ ID NO:7. This DNA sequence contains noncoding flanking regions at both the 3' and 5' ends. Analysis of this DNA sequence revealed a single long open reading frame that encodes a secretory signal peptide and the mature 3-hydroxysteroid oxidase protein of 43 and 504 amino acids, respectively. It is 84.37% identical to the published 3-hydroxysteroid oxidase nucleotide sequence. The derived amino acid sequence is 81.685% identical to the published 3-hydroxysteroid oxidase sequence. It is identified as SEQ ID NO: 8. Examination of the A19249 DNA sequence and comparison to the N-terminal amino acid sequence of intact 3-hydroxysteroid oxidase from A19249 revealed that the A19249 gene encoded a protein that includes a signal peptide sequence, which is apparently cleaved during secretion of the protein from the cells. Thus the N-terminus of the mature protein from A19249 begins with Ser-Gly-Gly-Thr-Phe, identified as SEQ ID NO:12.

## GENETIC TRANSFORMATION

A 3-hydroxysteroid oxidase gene can be isolated from novel organisms or may be obtained from known sources, such as the *Rhodococcus* sp. described by Long et al., in WO 90 05,788. This gene may then be used to transform bacterial cells or plant cells to enable the production of 3-hydroxysteroid oxidase and carry out methods of this invention. Examples of how this may be done with the gene of A19249 are given below.

Mutagenesis of the A19249 Gene

In order to incorporate the A19249 gene into vectors appropriate for expression of the 3-hydroxysteroid oxidase in heterologous bacterial or plant hosts, it was necessary to introduce appropriate restriction sites near the ends of the gene. The goals of this mutagenesis were to create cassettes that included the protein coding sequence with minimal non-coding flanking sequences and to incorporate useful restriction sites to mobilize these cassettes. Cassettes were designed that would allow mobilization of the intact coding sequence including the signal peptide or just the mature coding sequence. To incorporate these cassettes into appropriate bacterial or plant expression vectors, an NcoI restriction site was engineered at the N-terminus of the intact protein sequence or at the N-terminus of the mature protein sequence. A BamHI site was engineered just after the termination codon of the intact coding sequence. Three mutagenesis primers were designed to create these cassettes, as shown below. Mutagenesis with primer Chossn (SEQ ID NO:9) substituted three amino acids (MAT) for valine and asparagine at the N-terminus of the signal peptide of the intact protein and Chomnr (SEQ ID NO:10) added two amino acids (MA) at the N-terminus of the mature protein. This was necessary to allow incorporation of the NcoI restriction site. Mutagenesis with primer Cho3br (SEQ ID NO:11) incorporated a BamHI site at the 3' end of the coding sequence. Primers Chomnr and Cho3br were used to direct formation of the antisense strand of DNA.

Chossn (SEQ ID NO:9): CTCAGGAGCACCATGGCGACCGCACAC (NcoI site underlined)
Chomnr (SEQ ID NO:10):GTGCCGCCGGAGGCCATGGGGGCGGTGGC (NcoI site underlined)
Cho3br (SEQ ID NO:11): GCCCCGCCCGTCGGATCCGTCAGGAACCCG (BamHI site underlined)

The resulting modified sequences were identified as SEQ ID NO:13 encoding for the intact protein and SEQ ID NO:14 for the mature protein.

Expression of 3-Hyroxysteroid Oxidase in *E. coli*

The NcoI-BamHI fragments containing either the intact protein coding sequence or the mature protein coding sequence were inserted into a vector designed for protein expression in *E. coli*, vector pKK233-2 (Pharmacia LKB, Piscataway, NJ). pKK233-2 contains the IPTG-inducible trc promoter. The vector containing the intact (full length) protein coding sequence as modified (SEQ ID NO:13) is designated pMON20909. The vector containing the mature protein coding sequence as modified (SEQ ID NO:14) is designated pMON20907. *E. coli* XL1 Blue cells (Statagene, San Diego, CA) modified with pMON20909 expressed 3-hydroxysteroid oxidase at higher levels of enzymatic activity than cells modified with pMON20907. The protein was extracted and purified from 4 l of IPTG-induced *E. coli* containing pMON20909. The soluble fraction from sonicated bacterial lysate was concen-trated and dialyzed, and then partially purified by Mono Q chromatography to yield 11 units of 3-hydroxysteroid oxidase activity. Western blot analysis indicates that the signal sequence of the intact protein is cleaved in *E. coli*, but the exact site of cleavage was not determined. Analysis of the recovered protein showed a five-fold reduction in enzymatic activity relative to the A19249 protein, but the loss has not been explained by DNA sequencing which found no alterations that would explain loss of enzymatic activity in plant protoplasts or *E. coli.*

The recovered protein was tested against tobacco budworm and resulted in 88% stunting at a dose of 100 µg/ml.

Expression of 3-Hydroxysteroid Oxidase in Plant Colonizing Bacteria

To control insects, it may be desirable to express 3-hydroxysteroid oxidase in plant colonizing bacteria, and then apply this bacteria to the plant. As the insect feeds on the plant, it ingests a toxic dose of 3-hydroxysteroid oxidase produced by the plant colonizers. Plant colonizers can be either those that inhabit the plant surface, such as *Pseudomonas* or *Agrobacterium* species, or endophytes that inhabit the plant vasculature such as *Clavibacter* species. For surface colonizers, the 3-hydroxysteroid oxidase gene may be inserted into a broad host range vector capable of replicating in these Gram-negative hosts. Examples of these such vectors are pKT231 of the IncQ incompatibility group [Bagdasarian et al., 1981] or pVK100 of the IncP group [Knauf, 1982]. For endophytes the 3-hydroxysteroid oxidase gene can be inserted into the chromosome by homologous recombination or by incorporation of the gene onto an appropriate transposon capable of chromosomal insertion in these endophytic bacteria.

Plant Gene Construction

The expression of a plant gene which exists in double-stranded DNA form involves transcription of messenger RNA (mRNA) from one strand of the DNA by RNA polymerase enzyme, and the subsequent processing of the mRNA primary transcript inside the nucleus. This processing involves a 3' non-translated region which adds polyadenylate nucleotides to the 3' end of the RNA Transcription of DNA into mRNA is regulated by a region of DNA usually referred to as the "promoter." The promoter region contains a sequence of bases that signals RNA polymerase to associate with the DNA and to initiate the transcription of mRNA using one of the DNA strands as a template to make a corresponding strand of RNA.

A number of promoters which are active in plant cells have been described in the literature. Such promoters may be obtained from plants or plant viruses and include, but are not limited to, the nopaline synthase (NOS) and octopine synthase (OCS) promoters (which are carried on tumor-inducing plasmids of *Agrobacterium tumefaciens*), the cauliflower mosaic virus (CaMV) 19S and 35S promoters, the light-inducible promoter from the small subunit of ribulose 1,5-bisphosphate carboxylase (ssRUBISCO, a very abundant plant polypeptide), and the Figwort Mosaic Virus (FMV) 35S promoter. All of these promoters have been used to create various types of DNA constructs which have been expressed in plants (see e.g., PCT publication WO 84/02913).

The particular promoter selected should be capable of causing sufficient expression of the enzyme coding sequence to result in the production of an effective amount of 3-hydroxysteroid oxidase. A preferred promoter is a constitutive promoter such as FMV35S. It has been observed to provide more uniform expression of heterologous genes in the flowering portions of plants. Use of such a promoter with the 3-hydroxysteroid oxidase gene may provide greater protection of cotton bolls and squares from insect damage, than other promoters.

The promoters used in the DNA constructs (i.e. chimeric plant genes) of the present invention may be modified, if desired, to affect their control characteristics. For example, the CaMV35S promoter may be ligated to the portion of the ssRUBISCO gene that represses the expression of ssRUBISCO in the absence of light, to create a promoter which is active in leaves but not in roots. The resulting chimeric promoter may be used as described herein. For purposes of this description, the phrase "CaMV35S" promoter thus includes variations of CaMV35S promoter, e.g., promoters derived by means of ligation with operator regions, random or controlled mutagenesis, etc. Furthermore, the promoters may be altered to contain multiple "enhancer sequences" to assist in elevating gene expression. Examples of such enhancer sequences have been reported by Kay et al. (1987).

The RNA produced by a DNA construct of the present invention also contains a 5' non-translated leader sequence. This sequence can be derived from the promoter selected to express the gene, and can be specifically modified so as to increase translation of the mRNA. The 5' non-translated regions can also be obtained from viral RNA's, from suitable eukaryotic genes, or from a synthetic gene sequence. The present invention is not limited to constructs wherein the non-translated region is derived from the 5' non-translated sequence that accompanies the promoter sequence. As shown below, a plant gene leader sequence which is useful in the present invention is the petunia heat shock protein 70 (Hsp70) leader. [Winter et al.]

As noted above, the 3' non-translated region of the chimeric plant genes of the present invention contains a polyadenylation signal which functions in plants to cause the addition of adenylate nucleotides to the 3' end of the RNA. Examples of preferred 3' regions are (1) the 3' transcribed, non-translated regions containing the polyadenylate signal of *Agrobacterium* tumor-inducing (Ti) plasmid genes, such as the nopaline synthase (NOS) gene and (2) plant genes like the soybean 7s storage protein genes and the pea ssRUBISCO E9 gene. [Fischhoff et al.]

Plant Transformation and Expression

A chimeric plant gene containing a structural coding sequence of the present invention can be inserted into the genome of a plant by any suitable method. Suitable plant transformation vectors include those derived from a Ti plasmid of *Agrobacterium tumefaciens*, as well as those disclosed, e.g., by Herrera-Estrella (1983), Bevan (1983), Klee (1985) and EPO publication 0 120 516 (Schilperoort et al.). In addition to plant transformation vectors derived from the Ti or root-inducing (Ri) plasmids of *Agrobacterium*, alternative methods can be used to insert the DNA constructs of this invention into plant cells. Such methods may involve, for example, the use of liposomes, electroporation, chemicals that increase free DNA uptake, free DNA delivery via microprojectile bombardment, and transformation using viruses or pollen.

A particularly useful Ti plasmid cassette vector for transformation of dicotyledonous plants is pMON11782. The expression cassette pMON11782 consists of the FMV35S promoter, the petunia Hsp70 5' untranslated leader, and the 3' end including polyadenylation signals from the pea ssRUBISCO E9 gene. Between the leader and the 3' polyadenylation signals is a multilinker containing multiple restriction sites, including a BamHI site for the insertion of genes. pMON11782 also contains a HindIII site before the promoter sequence.

The remainder of pMON11782 contains a segment of pBR322 (New England Biolabs, Beverly, MA) which provides an origin of replication in *E. coli*; the oriV region from the broad host range plasmid RK1 which allows replication in *Agrobacterium* strain ABI; the streptomycinspectinomycin resistance gene from Tn7; and a chimeric NPTII gene, containing the CaMV35S promoter and the nopaline synthase (NOS) 3' end, which provides kanamycin resistance in transformed plant cells.

Another particularly useful Ti plasmid cassette vector is pMON17227. This vector is described by Barry et al. in WO 92/04449 and contains a gene encoding an enzyme conferring glyphosate resistance which is an excellent selection marker gene for many plants.

Transient Expression of 3-Hydroxysteroid Oxidase in Tobacco Plants

Both 3-hydroxysteroid oxidase gene cassettes, that is the gene encoding intact protein with the signal sequence and that encoding only the mature protein, each modified at the N-terminus as described above, were mobilized as NcoI-BamHI fragments and inserted into a transient expression vector that had been cut with NcoI and BamHI. A transient expression vector is a simple plasmid containing a plant promoter with a 5' nontranslated leader, a 3' nontranslated polyadenylation sequence, and between them a multilinker having multiple restriction sites for insertion of a protein coding sequence. The constructed vectors placed the 3-hydroxysteroid oxidase gene under the control of the FMV35S promoter with the petunia HSP70 leader sequence discussed above. At the 3' end terminator region is the non-translated polyadenylation signal terminator region of the nopaline synthase gene. A plasmid containing the intact protein coding sequence (SEQ ID NO:13) was identified and named pMON20910. A plasmid containing the modified mature protein coding sequence (SEQ ID NO:14) was identified and named pMON20908.

pMON20910 and pMON20908 are vectors for expression of 3-hydroxysteroid oxidase genes in plant cells, but these vectors lack appropriate sequences for use in *Agrobacterium*-mediated plant transformation. However, these vectors can be used for either transient expression of 3-hydroxysteroid oxidase in plant cells, or they can be used to generate stably transformed cotton plants via free DNA delivery such as biolistic bombardment of cotton meristems.

For transient expression analysis, plasmid DNA samples from pMON20908 and pMON20910 vectors were purified and introduced into tobacco via electroporation. Freeze-thaw extraction followed by a nine-fold concentration of soluble fractions on Centricon-10 filter concentrators allowed unambiguous detection of 3-hydroxysteroid oxidase activity in all cell lysates, immunologically by Western blot assay and enzymatically. The activity of the lysate from cells containing pMON20908, that is the coding sequence for the modified mature protein, was approximately ten-fold lower than that recovered from cells containing pMON20910. Western blot analysis indicated that the signal sequence is cleaved in protoplasts, although not necessarily with the fidelity necessary to generate a processed protein identical in form and activity to that naturally secreted by *Streptomyces* A19249.

<u>Stable Transformation of Dicots with a 3-Hydroxysteroid Oxidase Gene</u>

pMON20908 and pMON20910 were used to construct vectors for stable transformation of dicots with *Agrobacterium*. Each was partially digested with the restriction enzyme NotI to generate DNA fragments containing the FMV35S promoter, the petunia HSP70 leader, either the intact [full length] or mature 3-hydroxysteroid oxidase coding sequence, and nos 3' polyadenylation site. Partial digestion was necessary because in addition to NotI sites flanking the expression cassette, there are two NotI sites within the cholesterol oxidase gene - one within the coding region for the signal peptide and one within the coding region for the mature protein. The NotI partial digest fragments containing the entire expression cassettes are isolated using agarose gel electrophoresis and purified by extraction from the agarose. These NotI fragments are inserted into NotI-digested plasmid pMON17227, which is described by Barry et al. in WO 92/04449. pMON20913 was identified as containing the intact coding sequence. pMON20923 was identified as containing the mature coding sequence.

These vectors were introduced into disarmed *Agrobacterium* host ABI and used to transform tobacco in tissue culture. Selection for glyphosate resistance led to several transformed lines for each vector. For pMON20913, four out of 18 lines were confirmed as 3-hydroxysteroid oxidase expressors by Western blot assay. For pMON20923, four out of 29 lines were confirmed as 3-hydroxysteroid oxidase expressors by Western blot assay. Analysis of the lines transformed with pMON20913 confirm the presence of the 3-hydroxysteroid oxidase activity. The highest expressing plant had an expression level of 0.2% of total protein, which is equivalent to 54 ng of enzyme per mg of wet tissue.

Vectors containing the intact or mature 3-hydroxysteroid oxidase cassette express the active enzyme in the cytoplasm of the plant cell. There has been no evidence of secretion outside the transformed cells. Some bacterial secretory signal sequences have been shown to function in plant cells. It may be desirable to direct most or all of the 3-hydroxysteroid oxidase protein into the plant secretory pathway. To achieve this, it may be advantageous to use a signal sequence derived from a plant gene rather than a bacterial signal. An example of such a signal is that from the tobacco PR1b gene, described by Cornelissen et al. pMON 10824, disclosed in EP Publ. 0 385 962, is a plant transformation vector designed for the expression of the lepidopteran active *B.t. kurstaki* protein. In pMON10824, the *B.t.k.* coding sequence is fused to the PR1b signal sequence plus 10 amino acids of the mature PR1b coding sequence. This *B.t.k.* fusion gene is driven by the CaMV35S promoter containing a duplicated enhancer. Other vectors carrying the PR1B signal and CaMV35S promoter may also serve as the source of these elements. To create a vector in which the PR1b signal is fused to the 3-hydroxysteroid oxidase gene, a DNA fragment containing the CaMV35S promoter sequence and the PR1B signal sequence is used to replace the fragment containing the FMV promoter and HSP70 leader sequences in pMON 20913 and pMON20923. This results in plasmids in which the 3-hydroxysteroid oxidase coding sequence (either mature protein or intact protein cassette) is fused to the amino terminal secretory signal from the PR1b gene and is driven by the CaMV35S promoter. NcoI restriction enzyme sites at the 3' end of the PR1B signal-containing fragment and the 5' end of the modified 3-hydroxysteroid oxidase protein coding sequence allows in-frame translational fusions between the two elements. pMON20930 and pMON20932 are plant transformation vectors that carry such fusions between PR1B signal sequence and either the intact cholesterol oxidase and the mature cholesterol oxidase, respectively. A similar plasmid may be constructed wherein the 3-hydroxysteroid oxidase gene is under the control of the FMV35S promoter. Such plasmids are mobilized into a disarmed *Agrobacterium* host and used to transform dicots. Thus, these plants produce a 3-hydroxysteroid oxidase that is secreted into the extracellular space.

In some cases, proteins that enter the plant secretory pathway are targeted to different cellular compartments such as the vacuole. It may be desirable to direct the 3-hydroxysteroid oxidase to the vacuole of plant cells. In this case, the vectors described above in which the PR1b signal is used are further modified to include vacuolar targeting sequences derived from known plant vacuolar enzyme genes.

It may also be desirable to retain the 3-hydroxysteroid oxidase in the lumen of the endoplasmic reticulum (ER). In this case, vectors in which the PR1b signal is used to target the protein to the secretory pathway are further modified to include sequences known to encode ER retention signals. These sequences are added such that the four-amino acid-long ER retention peptides (such as HDEL (SEQ ID NO:15) and KDEL (SEQ ID NO:16) are fused to the carboxy terminus of the 3-hydroxysteroid oxidase. Site-directed oligonucleotide mutagenesis is used to introduce these additions to the carboxy-terminus coding sequence of 3-hydroxysteroid oxidase. pMON20937 and pMON20938 are vectors in which the peptides HDEL (SEQ ID NO:15) and RGSEKDEL (SEQ ID NO:17) are introduced, respectively, into pMON20932.

It may also be advantageous to direct the localization of the 3-hydroxysteroid oxidase protein to another cellular compartment, the chloroplast. Proteins can be directed to the chloroplast by including at their N-termini a chloroplast transit peptide (CTP). One CTP that has worked to localize heterologous proteins to the chloroplast is that derived from the RUBISCO small subunit gene of *Arabidopsis,* denoted ats1A. A variant of this transit peptide that encodes the transit peptide, 24 amino acids of mature RUBISCO sequence, plus a reiteration of the transit peptide cleavage site has been constructed for the successful chloroplast localization of the *B.t.k.* protein (Wong, 1992). Vectors containing the *Arabidopsis* ats1A transit peptide fused to the *B.t.k.* gene may be used as the base for constructing vectors for the chloroplast localization of the 3-hydroxysteroid oxidase protein. For example, pMON10817, constructed of the *Arabidopsis* RUBISCO small subunit promoter from the *ats1A* gene, the native *ats1A* 5' untranslated leader, the *ats1A* chloroplast transit peptide variant described above and a truncated *B.t.k.* enzyme gene is digested with restriction enzymes NotI and NcoI. A NotI - NcoI DNA fragment containing the promoter, leader and transit peptide is used to replace a NotI - NcoI fragment carrying the FMV promoter and HSP70 leader sequences in pMON20913 and pMON20923. These reactions construct plasmids pMON20929 and pMON20931 in which the 3-hydroxysteroid oxidase coding sequence (either intact protein or mature protein cassette, respectively) is fused at its amino terminus to the chloroplast transit peptide and is transcribed from the *ats1A* promoter. Alternatively, a similar plasmid may be constructed to replace the *ats1A* promoter with the CaMV35S or FMV35S promoters. Such plasmids are mobilized into disarmed *Agrobacterium* hosts and used to transform dicots. Thus, these plants produce a 3-hydroxysteroid oxidase that is localized to the chloroplast.

It may also be advantageous to direct the 3-hydroxysteroid oxidase to another subcellular compartment, the mitochondria. Examples of proteins that are normally localized to the mitochondria and for which the targeting sequences are known include cytochrome C1 which is localized to the mitochondrial intermembrane space (Braun, 1992) and the β subunit of ATP synthase which is localized to the mitochondrial matrix (Boutry, 1987). Mitochondrial targeting DNA sequences encoding the amino terminal mitochondrial targeting peptides are isolated using the polymerase chain reaction (PCR). Oligonucleotide primers corresponding to the amino and carboxy terminal portions of these targeting sequences are used to PCR-amplify first strand cDNA that has been generated from mRNA using reverse transcriptase. The oligonucleotide primers have NcoI restriction enzyme sites attached so that the amplified product, when cleaved with NcoI, can be inserted into the NcoI sites at the amino terminus of the cloned 3-hydroxysteroid oxidase gene cassettes. Thus, various forms of 3-hydroxysteroid oxidase fused to mitochondrial targeting peptides can be expressed in plants using promoters such as CaMV35S or FMV35S, and can be localized to the mitochondria.

Stable Transformation of Monocots

A 3-hydroxysteroid oxidase gene may be stably incorporated into the genome of monocots using the vectors and methods described in WO 93/19189 (Brown et al.). The gene can be inserted in an appropriate vector, for example pMON19653 and pMON19643, described by Brown et al. The resulting construct contains a cassette of the CaMV E35S promoter, the Hsp70 intron, the CP4 glyphosate selection marker, and the NOS terminator; a cassette of the CaMV E35S promoter, the Hsp70 intron, the GOX glyphosate selection marker, and the NOS terminator; and a single NotI site for insertion of a gene expression cassette containing a 3-hydroxysteroid oxidase gene, such as SEQ ID NO:13 or SEQ ID NO:14.

This vector is inserted by bombardment of embryogenic tissue culture cells using a biolistic particle gun as described by Brown et al. Transformed cells are selected for glyphosate resistance and whole plants are regenerated. Insect-resistant plants may be confirmed to be expressing the gene by Western blot analysis, esterase activity assay, or insect resistance assay.

Targeting of the protein to certain cellular compartments is also possible in monocots using the signal sequences described above.

From the foregoing, it will be seen that this invention is one well adapted to attain all the ends and objects hereinabove set forth together with advantages which are obvious and which are inherent to the invention. It will be understood that certain features and subcombinations are of utility and may be employed without reference to other features and subcombinations. This is contemplated by and is within the scope of the claims. Since many possible embodiments may be made of the invention without departing from the scope thereof, it is to be understood that all matter herein set forth or shown in the accompanying drawings is to be interpreted as illustrative and not in a limiting sense.

REFERENCES


Bagdasarian, M., et al. Gene, 16: 237-47, 1981.
Bevan, M. et al., Nature, 304:184, 1983.
Boutry, et al., Nature 328: 340-342, 1987.
Braun, et al., Mol. Gen. Genet. 231: 217-225, 1992.
Burnette, W.N. Anal. Biochem., 112: 195-203, 1981.
Cornelissen, B.J.C., et al. EMBO Journal, 5: 37-40, 1986.
Fischhoff, D.A and Perlak, F.J. "Synthetic plant genes and method for preparation." European Patent Application, Publication Number 0 385 962, 1990.
Gallo, L.L. Methods Enzymol., 71: 665-7, 1981.
Herrera-Estrella, L. et al., Nature, 303:209, 1983.
Ishizaki, T., et al. Journal of Bacteriology, 171: 596-601, 1989.
Kay, R. et al., Science, 236: 1299-1302, 1987.
Klee, H. J. et al., Bio/Technology, 3: 637-642, 1985.
Knauf, V.C. and Nester, E. Plasmid, 8: 43-54, 1982.
Laemmli, U.K Nature, 227: 680-5, 1970.
Long, Susan, and Ostroff, Gary R. PCT Int. Appl. WO 90 05,788.
Marrone, P.G., et al. J. Econ. Entom., 78: 290-3, 1985.
Matsudaira, P. J. Biol. Chem., 261: 10035-38, 1987.
Moore, S. and Stein, W.H. Methods in Enzymology, 6: 819-31, 1963.
Purcell, J.P., Greenplate, J.T., and Sammons, R.P. Insect Biochem. Molec. Biol., 22:41-47, 1992.
Purcell, J.P., et al. Biochem. Biophys. Res. Comm. 196:1406-1413, 1993.
Schuler, M. A. et al., Nucleic Acids Research, 10: 8225-8244, 1982.
Smith, A G., and Brooks, C.J.W. J. Steroid Biochem., 7: 705-713, 1976.
Smith, P.K, et al. Analytical Biochemistry, 150: 76-85, 1985.
Winter et al. Mol. Gen. Genet., 221(2): 315-19, 1988.
Wong, et al., Plant Mol. Biol. 20:81-93, 1992.
Yanisch-Perron, C., et al. Gene, 33: 103-19, 1985.

## Claims

1. A method of controlling lepidopteran insect infestation of plants comprising providing a 3-hydroxysteroid oxidase for ingestion by the insect.

2. The method of Claim 1 wherein the insect is in a larval stage.

3. The method of Claim 1 wherein said 3-hydroxysteroid oxidase is provided by plant-colonizing microorganisms which produce 3-hydroxysteroid oxidase after application to the plant.

4. The method of Claim 1 wherein said 3-hydroxysteroid oxidase is provided by expression of a gene for 3-hydroxysteroid oxidase incorporated in the plant by previous genetic transformation of a parent cell of the plant.

5. The method of Claim 4 wherein said plant is cotton or corn.

6. A method of producing a genetically transformed plant which expresses an amount of a 3-hydroxysteroid oxidase effective to control lepidopteran insect infestation, comprising the steps of:

   a) inserting into the genome of a plant cell a recombinant, double-stranded DNA molecule comprising

      (i) a promoter which functions in plant cells to cause the production of an RNA sequence;
      (ii) a structural coding sequence that encodes for 3-hydroxysteroid oxidase;
      (iii) a 3' non-translated region which functions in said plant cells to cause the addition of polyadenylate nucleotides to the 3' end of the RNA sequence,

   wherein said promoter is heterologous with respect to said structural coding sequence and wherein said promoter is operatively linked with said structural coding sequence, which is in turn operably linked with said non-translated region;
   b) obtaining transformed plant cells; and
   c) regenerating from the transformed plant cells genetically transformed plants with express an amount of 3-hydroxysteroid oxidase effective to control lepidopteran insect infestation.

7. The method of Claim 6 wherein said structural DNA sequence comprises SEQ ID NO:13 or SEQ ID NO: 14.

8. The method of Claim 6 wherein said plant cell is a cotton or corn plant cell.

9. The method of Claim 6 wherein wherein the genome of said plant cell also contains one or more genes expressing *B.t.* endotoxins.

## Patentansprüche

1. Verfahren zur Bekämpfung eines Schmetterlings-Insektenbefalls von Pflanzen, welches das Vorsehen von 3-Hydroxysteroidoxidase zur Aufnahme durch das Insekt umfaßt.

2. Verfahren nach Anspruch 1, bei welchem sich das Insekt im Larvenstadium befindet.

3. Verfahren nach Anspruch 1, bei welchem die 3-Hydroxysteroidoxidase von Pflanzen-kolonisierenden Mikroorganismen vorgesehen wird, die 3-Hydroxysteroidoxidase nach dem Aufbringen auf die Pflanze produzieren.

4. Verfahren nach Anspruch 1, bei welchem die 3-Hydroxysteroidoxidase durch die Expression eines Gens für 3-Hydroxysteroidoxidase vorgesehen wird, das in der Pflanze durch vorherige genetische Transformation einer Stammzelle der Pflanze eingebaut wird.

5. Verfahren nach Anspruch 4, bei welchem die Pflanze Baumwolle oder Mais ist.

6. Verfahren zur Herstellung einer genetisch transformierten Pflanze, die eine zur Bekämpfung eines Schmetterlings-Insektenbefalls wirksame Menge einer 3-Hydroxysteroidoxidase exprimiert, welches die Schritte umfaßt:

   a) Einfügen, in das Genom einer Pflanze, eines rekombinanten doppelsträngigen DNS-Moleküls, das umfaßt:

(i) einen Promotor, der in Pflanzenzellen wirksam ist, um die Produktion einer RNS-Sequenz zu verursachen;

(ii) eine strukturelle Codiersequenz, die für 3-Hydroxysteroidoxidase codiert,

(iii) eine 3'-nicht-translatierte Region, die in den Pflanzenzellen wirksam ist, um die Addition von Polyadenylatnucleotiden an das 3'-Ende der RNS-Sequenz zu verursachen;

wobei der Promotor heterolog ist in bezug auf die strukturelle Codiersequenz, und wobei der Promotor operativ mit der strukturellen Codiersequenz verbunden ist, die ihrerseits operativ mit der nicht-translatierten Region verbunden ist;

b) Erhalten transformierter Pflanzenzellen; und

c) Regenerieren, aus den transformierten Pflanzenzellen, genetisch transformierter Pflanzen, die eine zur Bekämpfung eines Schmetterlings-Insektenbefalls wirksame Menge an 3-Hydroxysteroidoxidase exprimieren.

7. Verfahren nach Anspruch 6, bei welchem die strukturelle DNS-Sequenz SEQ ID NO:13 oder SEQ ID NO:14 umfaßt.

8. Verfahren nach Anspruch 6, bei welchem die Pflanzenzelle eine Baumwoll- oder Mais-Pflanzenzelle ist.

9. Verfahren nach Anspruch 6, bei welchem das Genom der Pflanzenzelle auch eines oder mehrere Gene enthält, die B.t.-Endotoxine exprimieren.

**Revendications**

1. Procédé de lutte contre l'infestation de plantes par des insectes lépidoptères, qui comporte le fait d'apporter une 3-hydroxystéroïde oxydase pour qu'elle soit ingérée par les insectes.

2. Procédé conforme à la revendication 1, dans lequel les insectes se trouvent à l'état de larve.

3. Procédé conforme à la revendication 1, dans lequel la 3-hydroxystéroïde oxydase est apportée par des microorganismes qui colonisent la plante et qui produisent une 3-hydroxystéroïde oxydase après avoir été déposés sur la plante.

4. Procédé conforme à la revendication 1, dans lequel la 3-hydroxystéroïde oxydase est apportée par l'expression d'un gêne de 3-hydroxystéroïde oxydase, antérieurement incorporé dans la plante par transformation génétique d'une cellule parentale de la plante.

5. Procédé conforme à la revendication 4, dans lequel la plante est du coton ou du maïs.

6. Procédé de production de plantes génétiquement transformées qui produisent, par expression génétique, une 3-hydroxystéroïde oxydase en une quantité suffisante pour lutter efficacement contre l'infestation par des insectes lépidoptères, lequel procédé comporte les étapes suivantes :

a) insertion, dans le génome d'une cellule végétale, d'une molécule d'ADN double brin recombiné, comprenant

1) un promoteur dont le rôle, dans les cellules végétales, est de provoquer la production d'une séquence d'ARN,

2) une séquence codante de structure, qui code une 3-hydroxystéroïde oxydase, et

3) une région 3'-terminale non traduite dont le rôle, dans lesdites cellules végétales, est de provoquer l'addition d'une séquence poly(A) à l'extrémité 3'-terminale de la séquence d'ARN,

ledit promoteur étant hétérologue à ladite séquence codante de structure et opérationnellement lié à cette séquence codante de structure, laquelle est à son tour opérationnellement liée à ladite région non traduite ;

b) obtention de cellules végétales transformées ;

et c) régénération, à partir de ces cellules végétales transformées, de plantes génétiquement transformées qui produisent, par expression génétique, une 3-hydroxystéroïde oxydase en une quantité suffisante pour lutter efficacement contre l'infestation par des insectes lépidoptères.

7. Procédé conforme à la revendication 6, dans lequel ladite séquence d'ADN de structure comporte la Séquence n° 13 ou la Séquence n° 14.

8. Procédé conforme à la revendication 6, dans lequel ladite cellule végétale est une cellule de plant de coton ou de maïs.

9. Procédé conforme à la revendication 6, dans lequel le génome de ladite cellule végétale contient aussi un ou plusieurs gènes produisant des endotoxines de *Bacillus thuringiensis*.

SEQUENCE LISTING

(1) GENERAL INFORMATION:

(i) APPLICANT:
(A) NAME: Monsanto Company
(B) STREET: 800 North Lindbergh Boulevard
(C) CITY: St. Louis
(D) STATE: Missouri
(E) COUNTRY: United States of America
(F) POSTAL CODE (ZIP): 63167
(G) TELEPHONE: (314)694-3131
(H) TELEFAX: (314)694-5435

(ii) TITLE OF INVENTION: Method of Controlling Insects

(iii) NUMBER OF SEQUENCES: 14

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: US 07/762682
(B) FILING DATE: 23-SEP-1991

(vi) PRIOR APPLICATION DATA:
(A) APPLICATION NUMBER: US 07/937195
(B) FILING DATE: 09-SEP-1992

(vi) PRIOR APPLICATION DATA:
(A) APPLICATION NUMBER: US 08/083948
(B) FILING DATE: 28-JUN-1993

(2) INFORMATION FOR SEQ ID NO:1:

(i) SEQUENCE CHARACTERISTICS:
(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:1:

```
Val Ser Thr Leu Met Leu Glu Met Gly Gln Leu Trp Asn Gln Pro
1               5                   10                  15
```

(2) INFORMATION FOR SEQ ID NO:2:

(i) SEQUENCE CHARACTERISTICS:
(A) LENGTH: 16 amino acids
(B) TYPE: amino acid
(D) TOPOLOGY: linear

(ii) MOLECULE TYPE: peptide

(xi) SEQUENCE DESCRIPTION: SEQ ID NO:2:

Ala Phe Ala Asp Asp Phe Cys Tyr His Pro Leu Gly Gly Cys Val Leu
1 5 10 15

(2) INFORMATION FOR SEQ ID NO:3:

(i) SEQUENCE CHARACTERISTICS:
(A) LENGTH: 12 amino acids
(B) TYPE: amino acid
(D) TOPOLOGY: linear

(ii) MOLECULE TYPE: peptide

(xi) SEQUENCE DESCRIPTION: SEQ ID NO:3:

Asn Leu Tyr Val Thr Asp Gly Ser Leu Ile Pro Gly
1 5 10

(2) INFORMATION FOR SEQ ID NO:4:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

GTGTCCACCC TGATGCTGGA GATGGGCCAG CTGTGGAACC AGCCC 45

(2) INFORMATION FOR SEQ ID NO:5:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

GCCTTCGCCG ACGACTTCTG CTACCACCCG CTCGGCGGCT GCGTCCTG 48

(2) INFORMATION FOR SEQ ID NO:6:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:6:

AACCTCTACG TGACCGACGG TTCGCTGATC CCGGGT 36

(2) INFORMATION FOR SEQ ID NO:7:

(i) SEQUENCE CHARACTERISTICS:
(A) LENGTH: 1865 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:7:

CTACTCCATG GCGTGCTGAA GGTCGGTGCC TGGCCTCCCG AGGTCGTCGA GGACTTCGTG 60

AAGTGAGCGG GCACCCCGCC CGTCCCCGCC CCGCAACGGC CCGTTCCGCA CACCGGGTGA 120

CCCGACCCCC TCGGCCCCCG ACGTCCGCCG ACCTCTCAGT CCCCTCTCGA AGCTCAGGAG 180

CAACAGCGTG AACGCACACC AGCCTCTGTC GCGCCGCCGC ATGCTCGGCC TGGCCGCCTT 240

GGGCGCCGCC GCACTCACCG GGCAGACCAC GATCACCGCG GCCCCCCGCG CGGCCGCCGC 300

CACCGCCCCC GGCGGCTCCG GCGGCACGTT CGTGCCCGCC GTCGTGATCG GCACCGGCTA 360

CGGCGCGGCC GTCTCCGCCC TGCGGCTCGG CGAGGCCGGG GTCTCCACCC TGATGCTGGA 420

GATGGGCCAG CTGTGGAACC AGCCCGGCCC GGACGGCAAC GTCTTCTGCG GGATGCTCAA 480

GCCCGACAAG CGCTCCAGCT GGTTCAAGAC CCGCACCGAG GCCCCGCTCG GCTCCTTCCT 540

CTGGCTCGAC CTCGCCAACC GGGACATCGA CCCCTACGCG GGCGTCCTGG ACCGGGTCAA 600

CTTCGACCAG ATGTCCGTGT ACGTGGGCCG CGGGGTCGGC GGCGGCTCGC TCGTCAACGG 660

CGGTATGGCC GTCACGCCCC GGCGCTCCTA CTTCCAGGAG GTGCTGCCCC AGGTCGACGC 720

CGACGAGATG TACGGCACCT ACTTCCCGCG CGCGAACTCC GGCCTGCGGG TCAACAACAT 780

CGACAAGGAC TGGTTCGAGC AGACCGAGTG GTACACGTTC GCGCGCGTTG CCCGTCTGCA 840

GGCCGAGAAC GCCGGCCTGA AGACCACCTT CGTGCCCAAC GTCTACGACT GGGACTACAT 900

GCGCGGTGAG GCGGACGGCA CCAACCCCAA GTCCGCGCTC GCCGCCGAGG TCATCTACGG 960

CAACAACCAC GGCAAGGTCT CCCTCGACAA GAGCTACCTG GCGGCCGCCC TGGGCACCGG 1020

CAAGGTCACC GTCGAGACCC TGCACCAGGT CAAGACGATC CGTCAGCAGA ACGACGGCAC 1080

CTACCTGCTG ACGGTCGAGC AGAAGGACCC CGACGGCAAG CTGCTCGGGA CCAAGGAGAT 1140

CTCCTGCCGC CACCTCTTCC TCGGCGCCGG CAGCCTCGGC TCCATTGAAC TGCTGCTGCG 1200

CGCCCGGGAG ACCGGCACCC TGCCCGGCCT CAGCTCCGAG ATCGGCGGCG GCTGGGGCCC 1260

CAACGGCAAC ATCATGACCG CCCGCGCCAA CCATGTGTGG AACCCCACGG GCAGCAAGCA 1320

GTCGTCGATC CCCGCCCTCG GCATCGACGA CTGGGACAAC CCCGACAACC CCGTCTTCGC 1380

```
CGAGATAGCC CCCATGCCGG CGGGCCTCGA GACCTGGGTC AGCCTCTACC TGGCCATCAC 1440

CAAGAACCCG GAGCGCGGCA CCTTCGTCTA CGACGCCGCC AAGGACCGGG CGGACCTGCG 1500

CTGGACCCGG GACCAGAACG CGCCCGCGGT CGCCGCCGCC AAGTCGCTGT TCGACCGCGT 1560

CAACAAGGCC AACACGACCA TCTACCGGTA CGACCTCTTC GGCAAGCAGA TCAAGGCGTT 1620

CGCCGACGAC TTCTGCTACC ACCCGCTCGG CGGCTGCGTC CTCGGCAAGG CCACCGACAA 1680

CTACGGCCGC GTCTCCGGGT ACAAGAACCT CTACGTCACC GACGGCTCGC TCATCCCCGG 1740

CAGCATCGGC GTCAACCCGT TCGTGACCAT CACGGCGCTG GCGGAGCGGA ACGTCGAGCG 1800

CGTCATCAAG GAGGACATCG CGGGTTCCTG ACGAGCGACG GGCGGGGCGC GGCATGCAAG 1860

CTTGG                                                             1865
```

(2) INFORMATION FOR SEQ ID NO:8:

(i) SEQUENCE CHARACTERISTICS:
(A) LENGTH: 547 amino acids
(B) TYPE: amino acid
(D) TOPOLOGY: linear

(ii) MOLECULE TYPE: peptide

(xi) SEQUENCE DESCRIPTION: SEQ ID NO:8:

```
Val Asn Ala His Gln Pro Leu Ser Arg Arg Arg Met Leu Gly Leu Ala
1               5                   10                  15

Ala Leu Gly Ala Ala Ala Leu Thr Gly Gln Thr Thr Ile Thr Ala Ala
            20                  25                  30

Pro Arg Ala Ala Ala Ala Thr Ala Pro Gly Gly Ser Gly Gly Thr Phe
        35                  40                  45

Val Pro Ala Val Val Ile Gly Thr Gly Tyr Gly Ala Ala Val Ser Ala
    50                  55                  60

Leu Arg Leu Gly Glu Ala Gly Val Ser Thr Leu Met Leu Glu Met Gly
65                  70                  75                  80

Gln Leu Trp Asn Gln Pro Gly Pro Asp Gly Asn Val Phe Cys Gly Met
                85                  90                  95

Leu Lys Pro Asp Lys Arg Ser Ser Trp Phe Lys Thr Arg Thr Glu Ala
            100                 105                 110

Pro Leu Gly Ser Phe Leu Trp Leu Asp Leu Ala Asn Arg Asp Ile Asp
        115                 120                 125

Pro Tyr Ala Gly Val Leu Asp Arg Val Asn Phe Asp Gln Met Ser Val
    130                 135                 140

Tyr Val Gly Arg Gly Val Gly Gly Gly Ser Leu Val Asn Gly Gly Met
145                 150                 155                 160

Ala Val Thr Pro Arg Arg Ser Tyr Phe Gln Glu Val Leu Pro Gln Val
```

165 170 175

Asp Ala Asp Glu Met Tyr Gly Thr Tyr Phe Pro Arg Ala Asn Ser Gly
180 185 190

Leu Arg Val Asn Asn Ile Asp Lys Asp Trp Phe Glu Gln Thr Glu Trp
195 200 205

Tyr Thr Phe Ala Arg Val Ala Arg Leu Gln Ala Glu Asn Ala Gly Leu
210 215 220

Lys Thr Thr Phe Val Pro Asn Val Tyr Asp Trp Asp Tyr Met Arg Gly
225 230 235 240

Glu Ala Asp Gly Thr Asn Pro Lys Ser Ala Leu Ala Ala Glu Val Ile
245 250 255

Tyr Gly Asn Asn His Gly Lys Val Ser Leu Asp Lys Ser Tyr Leu Ala
260 265 270

Ala Ala Leu Gly Thr Gly Lys Val Thr Val Glu Thr Leu His Gln Val
275 280 285

Lys Thr Ile Arg Gln Gln Asn Asp Gly Thr Tyr Leu Leu Thr Val Glu
290 295 300

Gln Lys Asp Pro Asp Gly Lys Leu Leu Gly Thr Lys Glu Ile Ser Cys
305 310 315 320

Arg His Leu Phe Leu Gly Ala Gly Ser Leu Gly Ser Ile Glu Leu Leu
325 330 335

Leu Arg Ala Arg Glu Thr Gly Thr Leu Pro Gly Leu Ser Ser Glu Ile
340 345 350

Gly Gly Gly Trp Gly Pro Asn Gly Asn Ile Met Thr Ala Arg Ala Asn
355 360 365

His Val Trp Asn Pro Thr Gly Ser Lys Gln Ser Ser Ile Pro Ala Leu
370 375 380

Gly Ile Asp Asp Trp Asp Asn Pro Asp Asn Pro Val Phe Ala Glu Ile
385 390 395 400

Ala Pro Met Pro Ala Gly Leu Glu Thr Trp Val Ser Leu Tyr Leu Ala
405 410 415

Ile Thr Lys Asn Pro Glu Arg Gly Thr Phe Val Tyr Asp Ala Ala Lys
420 425 430

Asp Arg Ala Asp Leu Arg Trp Thr Arg Asp Gln Asn Ala Pro Ala Val
435 440 445

Ala Ala Ala Lys Ser Leu Phe Asp Arg Val Asn Lys Ala Asn Thr Thr
450 455 460

Ile Tyr Arg Tyr Asp Leu Phe Gly Lys Gln Ile Lys Ala Phe Ala Asp
465 470 475 480

Asp Phe Cys Tyr His Pro Leu Gly Gly Cys Val Leu Gly Lys Ala Thr
485 490 495

```
Asp Asn Tyr Gly Arg Val Ser Gly Tyr Lys Asn Leu Tyr Val Thr Asp
            500                 505                 510

Gly Ser Leu Ile Pro Gly Ser Ile Gly Val Asn Pro Phe Val Thr Ile
        515                 520                 525

Thr Ala Leu Ala Glu Arg Asn Val Glu Arg Val Ile Lys Glu Asp Ile
    530                 535                 540

Ala Gly Ser
545
```

(2) INFORMATION FOR SEQ ID NO:9:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:9:

CTCAGGAGCA CCATGGCGAC CGCACAC 27

(2) INFORMATION FOR SEQ ID NO:10:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:10:

GTGCCGCCGG AGGCCATGGG GGCGGTGGC 29

(2) INFORMATION FOR SEQ ID NO:11:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

GCCCCGCCCG TCGGATCCGT CAGGAACCCG 30

(2) INFORMATION FOR SEQ ID NO:12:

(i) SEQUENCE CHARACTERISTICS:
(A) LENGTH: 5 amino acids

(B) TYPE: amino acid
(D) TOPOLOGY: linear

(ii) MOLECULE TYPE: peptide

(xi) SEQUENCE DESCRIPTION: SEQ ID NO:12:

Ser Gly Gly Thr Phe
1 5

(2) INFORMATION FOR SEQ ID NO:13:

(i) SEQUENCE CHARACTERISTICS:
(A) LENGTH: 1647 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

ATGGCGACCG CACACCAGCC TCTGTCGCGC CGCCGCATGC TCGGCCTGGC CGCCTTGGGC 60

GCCGCCGCAC TCACCGGGCA GACCACGATC ACCGCGGCCC CCCGCGCGGC CGCCGCCACC 120

GCCCCCGGCG GCTCCGGCGG CACGTTCGTG CCCGCCGTCG TGATCGGCAC CGGCTACGGC 180

GCGGCCGTCT CCGCCCTGCG GCTCGGCGAG GCCGGGGTCT CCACCCTGAT GCTGGAGATG 240

GGCCAGCTGT GGAACCAGCC CGGCCCGGAC GGCAACGTCT TCTGCGGGAT GCTCAAGCCC 300

GACAAGCGCT CCAGCTGGTT CAAGACCCGC ACCGAGGCCC CGCTCGGCTC CTTCCTCTGG 360

CTCGACCTCG CCAACCGGGA CATCGACCCC TACGCGGGCG TCCTGGACCG GGTCAACTTC 420

GACCAGATGT CCGTGTACGT GGGCCGCGGG GTCGGCGGCG GCTCGCTCGT CAACGGCGGT 480

ATGGCCGTCA CGCCCCGGCG CTCCTACTTC CAGGAGGTGC TGCCCCAGGT CGACGCCGAC 540

GAGATGTACG GCACCTACTT CCCGCGCGCG AACTCCGGCC TGCGGGTCAA CAACATCGAC 600

AAGGACTGGT TCGAGCAGAC CGAGTGGTAC ACGTTCGCGC GCGTTGCCCG TCTGCAGGCC 660

GAGAACGCCG GCCTGAAGAC CACCTTCGTG CCCAACGTCT ACGACTGGGA CTACATGCGC 720

GGTGAGGCGG ACGGCACCAA CCCCAAGTCC GCGCTCGCCG CCGAGGTCAT CTACGGCAAC 780

AACCACGGCA AGGTCTCCCT CGACAAGAGC TACCTGGCGG CCGCCCTGGG CACCGGCAAG 840

GTCACCGTCG AGACCCTGCA CCAGGTCAAG ACGATCCGTC AGCAGAACGA CGGCACCTAC 900

CTGCTGACGG TCGAGCAGAA GGACCCCGAC GGCAAGCTGC TCGGGACCAA GGAGATCTCC 960

TGCCGCCACC TCTTCCTCGG CGCCGGCAGC CTCGGCTCCA TTGAACTGCT GCTGCGCGCC 1020

CGGGAGACCG GCACCCTGCC CGGCCTCAGC TCCGAGATCG GCGGCGGCTG GGGCCCCAAC 1080

GGCAACATCA TGACCGCCCG CGCCAACCAT GTGTGGAACC CCACGGGCAG CAAGCAGTCG 1140

TCGATCCCCG CCCTCGGCAT CGACGACTGG GACAACCCCG ACAACCCCGT CTTCGCCGAG 1200

ATAGCCCCCA TGCCGGCGGG CCTCGAGACC TGGGTCAGCC TCTACCTGGC CATCACCAAG 1260

AACCCGGAGC GCGGCACCTT CGTCTACGAC GCCGCCAAGG ACCGGGCGGA CCTGCGCTGG 1320

ACCCGGGACC AGAACGCGCC CGCGGTCGCC GCCGCCAAGT CGCTGTTCGA CCGCGTCAAC 1380

AAGGCCAACA CGACCATCTA CCGGTACGAC CTCTTCGGCA AGCAGATCAA GGCGTTCGCC 1440

GACGACTTCT GCTACCACCC GCTCGGCGGC TGCGTCCTCG GCAAGGCCAC CGACAACTAC 1500

GGCCGCGTCT CCGGGTACAA GAACCTCTAC GTCACCGACG GCTCGCTCAT CCCCGGCAGC 1560

ATCGGCGTCA ACCCGTTCGT GACCATCACG GCGCTGGCGG AGCGGAACGT CGAGCGCGTC 1620

ATCAAGGAGG ACATCGCGGG TTCCTGA 1647

(2) INFORMATION FOR SEQ ID NO:14:

(i) SEQUENCE CHARACTERISTICS:
(A) LENGTH: 1521 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:14:

ATGGCCTCCG GCGGCACGTT CGTGCCCGCC GTCGTGATCG GCACCGGCTA CGGCGCGGCC 60

GTCTCCGCCC TGCGGCTCGG CGAGGCCGGG GTCTCCACCC TGATGCTGGA GATGGGCCAG 120

CTGTGGAACC AGCCCGGCCC GGACGGCAAC GTCTTCTGCG GGATGCTCAA GCCCGACAAG 180

CGCTCCAGCT GGTTCAAGAC CCGCACCGAG GCCCCGCTCG GCTCCTTCCT CTGGCTCGAC 240

CTCGCCAACC GGGACATCGA CCCCTACGCG GGCGTCCTGG ACCGGGTCAA CTTCGACCAG 300

ATGTCCGTGT ACGTGGGCCG CGGGGTCGGC GGCGGCTCGC TCGTCAACGG CGGTATGGCC 360

GTCACGCCCC GGCGCTCCTA CTTCCAGGAG GTGCTGCCCC AGGTCGACGC CGACGAGATG 420

TACGGCACCT ACTTCCCGCG CGCGAACTCC GGCCTGCGGG TCAACAACAT CGACAAGGAC 480

TGGTTCGAGC AGACCGAGTG GTACACGTTC GCGCGCGTTG CCCGTCTGCA GGCCGAGAAC 540

GCCGGCCTGA AGACCACCTT CGTGCCCAAC GTCTACGACT GGGACTACAT GCGCGGTGAG 600

GCGGACGGCA CCAACCCCAA GTCCGCGCTC GCCGCCGAGG TCATCTACGG CAACAACCAC 660

GGCAAGGTCT CCCTCGACAA GAGCTACCTG GCGGCCGCCC TGGGCACCGG CAAGGTCACC 720

GTCGAGACCC TGCACCAGGT CAAGACGATC CGTCAGCAGA ACGACGGCAC CTACCTGCTG 780

ACGGTCGAGC AGAAGGACCC CGACGGCAAG CTGCTCGGGA CCAAGGAGAT CTCCTGCCGC 840

CACCTCTTCC TCGGCGCCGG CAGCCTCGGC TCCATTGAAC TGCTGCTGCG CGCCCGGGAG 900

ACCGGCACCC TGCCCGGCCT CAGCTCCGAG ATCGGCGGCG GCTGGGGCCC CAACGGCAAC 960

```
ATCATGACCG CCCGCGCCAA CCATGTGTGG AACCCCACGG GCAGCAAGCA GTCGTCGATC 1020
CCCGCCCTCG GCATCGACGA CTGGGACAAC CCCGACAACC CCGTCTTCGC CGAGATAGCC 1080
CCCATGCCGG CGGGCCTCGA GACCTGGGTC AGCCTCTACC TGGCCATCAC CAAGAACCCG 1140
GAGCGCGGCA CCTTCGTCTA CGACGCCGCC AAGGACCGGG CGGACCTGCG CTGGACCCGG 1200
GACCAGAACG CGCCCGCGGT CGCCGCCGCC AAGTCGCTGT TCGACCGCGT CAACAAGGCC 1260
AACACGACCA TCTACCGGTA CGACCTCTTC GGCAAGCAGA TCAAGGCGTT CGCCGACGAC 1320
TTCTGCTACC ACCCGCTCGG CGGCTGCGTC CTCGGCAAGG CCACCGACAA CTACGGCCGC 1380
GTCTCCGGGT ACAAGAACCT CTACGTCACC GACGGCTCGC TCATCCCCGG CAGCATCGGC 1440
GTCAACCCGT TCGTGACCAT CACGGCGCTG GCGGAGCGGA ACGTCGAGCG CGTCATCAAG 1500
GAGGACATCG CGGGTTCCTG A                                           1521
```

(2) INFORMATION FOR SEQ ID NO:15:

(i) SEQUENCE CHARACTERISTICS:
(A) LENGTH: 4 amino acids
(B) TYPE: amino acid
(D) TOPOLOGY: linear

(ii) MOLECULE TYPE: peptide

(xi) SEQUENCE DESCRIPTION: SEQ ID NO:15:

```
His Asp Glu Leu
1
```

(2) INFORMATION FOR SEQ ID NO:16:

(i) SEQUENCE CHARACTERISTICS:
(A) LENGTH: 4 amino acids
(B) TYPE: amino acid
(D) TOPOLOGY: linear

(ii) MOLECULE TYPE: peptide

(xi) SEQUENCE DESCRIPTION: SEQ ID NO:16:

```
Lys Asp Glu Leu
1
```

(2) INFORMATION FOR SEQ ID NO:17:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:17:

```
Arg Gly Ser Glu Lys Asp Glu Leu
1               5
```